# EUROPEAN PATENT APPLICATION

(11) **EP 4 039 267 A1**
(43) Date of publication of application: **10.08.2022**
(21) Application number: 20885467.9
(22) Date of filing: 06.11.2020
(51) Int. Cl.: A61K 38/17, A61K 31/4422, A61P 25/28, A61P 25/16

(54) **COMPOSITION, COMPRISING TMEM176B OR AN EXPRESSION OR ACTIVITY REGULATOR THEREOF AS AN ACTIVE INGREDIENT, FOR PREVENTION OR TREATMENT OF DEGENERATIVE BRAIN DISEASE**

(30) Priority: 07.11.2019 KR 20190141927
(71) Applicant: Amyloid Solution Inc., Seongnam-si, Gyeonggi-do 13486 (KR)
(72) Inventor: KIM, Junghwan, Yongin-si Gyeonggi-do 16941 (KR); PARK, Sanghoon, Seongnam-si Gyeonggi-do 13458 (KR); PARK, Seongjeong, Yongin-si Gyeonggi-do 16874 (KR); KIM, Hye-Ju, Hwaseong-si Gyeonggi-do 18444 (KR)
(74) Representative: Vossius & Partner Patentanwälte Rechtsanwälte mbB
(86) International application number: PCT/KR2020/015530
(87) International publication number: WO 2021/091316

(57) **Abstract**

Provided is a composition for prophylaxis or treatment of neurodegenerative brain disease, the composition including TMEM176B as an active ingredient. The composition according to an aspect not only promotes phagocytosis of amyloid-beta in astrocytes and reduces amyloid plaques, but also exhibits an effect of ameliorating behavior and cognitive functions in an animal model having Alzheimer's disease. Therefore, the composition can be usefully utilized for the prophylaxis and/or treatment of various neurodegenerative brain diseases.

<110>    Amyloid Solution Inc.

<120>    Composition for preventing or treating neurodegenerative brain
         diseases comprising TMEM176B, its expression or activity
         modulator as an active ingredient

<130>    PX202064

<150>    KR 10-2019-0141927
<151>    2019-11-07

<160>    1

<170>    KoPatentIn 3.0

<210>    1
<211>    131
<212>    PRT
<213>    Artificial Sequence

<220>
<223>    amino acid sequence of TMEM176B


<400>    1
Met Ala Ala Val Val Leu Cys Val Asn Ser Phe Ile Trp Gln Thr Glu
  1               5                  10                  15

Pro Phe Leu Tyr Ile Asp Thr Val Cys Asp Arg Ser Asp Pro Val Phe
             20                  25                  30

Pro Thr Thr Gly Tyr Arg Trp Met Arg Arg Ser Gln Glu Asn Gln Trp
         35                  40                  45

Gln Lys Glu Glu Cys Arg Ala Tyr Met Gln Met Leu Arg Lys Leu Phe
     50                  55                  60

Thr Ala Ile Arg Ala Leu Phe Leu Ala Val Cys Val Leu Lys Val Ile
 65                  70                  75                  80

Val Ser Leu Val Ser Leu Gly Val Gly Leu Arg Asn Leu Cys Gly Gln
                 85                  90                  95

Ser Ser Gln Pro Leu Asn Glu Glu Gly Ser Glu Lys Arg Leu Leu Gly
            100                 105                 110

Glu Asn Ser Val Pro Pro Ser Pro Ser Arg Glu Gln Thr Ser Thr Ala
        115                 120                 125

Ile Val Leu
    130

## Description

### TECHNICAL FIELD

The present disclosure relates to a composition for preventing or treating neurodegenerative brain disease.

### BACKGROUND ART

Neurodegenerative brain disease is a disease causing various symptoms such as inhibition of high-dimensional causal functions including impairment of motor and sensory functions, memory, learning, arithmetic reasoning, and the like, as neurodegenerative changes appear in neurons of the central nervous system. Types of neurodegenerative brain disease include Alzheimer's disease, Parkinson's disease, and memory disorder. Neurodegenerative brain disease is characterized by death of neurons by necrosis or apoptosis that occurs rapidly or slowly. Therefore, understanding of the neuronal apoptosis mechanism should be made for the development of prevention, control, and treatment of the central nervous system diseases.

Meanwhile, an important pathological feature of Alzheimer's, which is a type of neurodegenerative brain disease, is formation of peptide aggregates called senile plaques, wherein the senile plaques cause synaptic dysfunction and apoptosis of neurons. One of components of the senile plaques is amyloid-beta, which is 40 to 42 amino acids long. Amyloid-beta monomers are easy to self-assemble into oligomers, protofibrils, fibers rich in beta-sheets, and are related to the pathogenesis of neurotoxicity.

Although the correlation between amyloid-beta plaques and neurotoxicity has not yet been clearly defined, the self-assembly of amyloid-beta into intermediate oligomers or aggregates is considered to be related to the development of cranial nerve diseases such as Alzheimer's disease.

Meanwhile, it has been recently reported that glial cells and microglial cells have phagocytosis activity on can phagocytize amyloid-beta, which is a peptide as a major cause of Alzheimer's disease (Chung, WS, et al., Do glia drive synaptic and cognitive impairment in disease?, Nat Neurosci, 2015.18(11): p.1539-45.)

Therefore, inhibiting aggregation of amyloid-beta or disaggregating aggregates of amyloid-beta, reducing tau proteins that are abnormally hyperphosphorylated, or increasing phagocytosis activity of glial cells is suggested as a method of treating neurodegenerative brain disease such as Alzheimer's disease and Parkinson's disease.

### DESCRIPTION OF EMBODIMENTS

### TECHNICAL PROBLEM

An aspect provides a pharmaceutical composition for preventing or treating neurodegenerative brain disease, including, as an active ingredient, transmembrane protein 176B (TMEM176B), a polynucleotide encoding the same, or an agent for enhancing activity or expression of the TMEM176B or the polynucleotide.

Another aspect provides a pharmaceutical composition for preventing or treating neurodegenerative brain disease, including, as an active ingredient, a vector including a polynucleotide encoding transmembrane protein 176B (TMEM176B).

Another aspect provides a pharmaceutical composition for preventing or treating neurodegenerative brain disease, including, as an active ingredient, a cell transformed with a vector that includes a polynucleotide encoding transmembrane protein 176B (TMEM176B).

Another aspect provides a functional health food for preventing or ameliorating neurodegenerative brain disease, including, as an active ingredient, an agent for enhancing activity or expression of transmembrane protein 176B (TMEM176B) or a polynucleotide encoding the same.

Another aspect provides a functional health food for preventing or ameliorating cognitive impairment or improving memory, including, as an active ingredient, an agent for enhancing activity or expression of transmembrane protein 176B (TMEM176B) or a polynucleotide encoding the same.

Another aspect provides a method of preventing, ameliorating, or treating neurodegenerative brain disease, the method including administering an effective amount of transmembrane protein 176B (TMEM176B), a polynucleotide encoding the same, or an agent for enhancing activity or expression of the TMEM176B or the polynucleotide encoding the same, to a subject in need of the TMEM176B, the polynucleotide, or the agent.

Another aspect provides use of an effective amount of transmembrane protein 176B (TMEM176B), a polynucleotide encoding the same, or an agent for enhancing activity or expression of the TMEM176B or the polynucleotide for preparation of a composition for preventing, ameliorating, or treating neurodegenerative brain disease.

### SOLUTION TO PROBLEM

An aspect provides a pharmaceutical composition for preventing or treating neurodegenerative brain disease, including, as an active ingredient, transmembrane protein 176B (TMEM176B), a polynucleotide encoding the same, or an agent for enhancing activity or expression of the TMEM176B or the polynucleotide.

In the present specification, the term "transmembrane protein 176B (TMEM176B)" may refer to a protein having a transmembrane region, and is also referred to as LR8 or MS4B2. In the present specification, the TMEM176B may refer to all of paralogs, orthologs, or homologs of the TMEM176B. In an embodiment, the TMEM176B may include an amino acid sequence of SEQ ID NO: 1.

In the present specification, the term "polynucleotide" may refer to a single-stranded or double-stranded polymer of deoxyribonucleotides or ribonucleotides. The polynucleotide may include an RNA genome sequence, DNA (gDNAs and cDNAs), and an RNA sequences transcribed therefrom, and unless defined otherwise, may include an analog of a natural polynucleotide.

The polynucleotide may include not only a nucleotide sequence encoding the amino acid sequence of the TMEM176B, but also a complementary sequence to the nucleotide sequence. The complementary sequence may include not only perfectly complementary sequences, but also substantially complementary sequences. Under the stringent conditions known in the art, the complementary sequence may refer to, for example, a sequence capable of hybridizing with a nucleotide sequence of the nucleotide sequence encoding the amino acid sequence of the protein.

The agent for enhancing activity or expression may refer to a substance that improves, induces, stimulates, or increases the expression or activity of the TMEM176B by acting thereon directly or indirectly. Examples of the substance are a single compound, such as an organic or inorganic compound, a biopolymer, such as a peptide, a protein, an antibody, a nucleic acid, a carbohydrate, and a lipid, and a composite of multiple compounds. A mechanism in which the substance enhances (promotes) the activity or expression of the TMEM176B is not particularly limited. For example, the mechanism may include a case where the expression including transcription and translation of a gene is enhanced.

In an embodiment, the pharmaceutical composition may be administered to:
(1) a subject in which a level of amyloid-beta or an aggregation level of amyloid-beta is higher than or at risk of being higher than normal not having neurodegenerative brain disease;
(2) a subject in which an aggregation level of tau protein is higher than or at risk of being higher than normal not having neurodegenerative brain disease;
(3) a subject in which a phosphorylation level of tau protein is higher than or at risk of being higher than normal not having neurodegenerative brain disease;
(4) a subject in which functions of glial cells are more dysfunctional than or at risk of being more dysfunctional than normal not having neurodegenerative brain disease; and
(5) a subject corresponding to one or more of (1) to (4).

The aggregation level of amyloid-beta or tau proteins may refer to the amount (concentration) of the amyloid-beta aggregates or the tau proteins aggregates, or a ratio of the amount (concentration) of amyloid-beta aggregates or the tau protein aggregates to the total amount (concentration) of amyloid-beta or total amount (concentration) of tau protein.

The phosphorylation level of tau protein may refer to the amount (concentration) of the phosphorylated tau protein or a ratio of the amount (concentration) of phosphorylated tau proteins to the total amount (concentration) of tau protein.

Human amyloid-beta is a peptide molecule including about 36 to 43 amino acids. The amyloid-beta is a major component of amyloid plaques expressed in the brain of a patient with Alzheimer's disease, and is known to be involved in the pathogenesis of Alzheimer's disease. The amyloid-beta peptide molecule may be obtained by cleaving amyloid precursor protein (APP; UniProtKB P05067) with beta secretase and gamma secretase. The amyloid-beta peptide molecule may be aggregated to form neurotoxic oligomers, thereby causing neurodegenerative brain disease.

The tau protein may consist of four parts: an N-terminal protruding part, a proline aggregation domain, a micro-organelle-binding domain, and a C-terminus (Mandelkow et al., Acta. Neuropathol., 103, 26-35, 1996). The tau protein is known to cause neurodegenerative brain disease, such as Parkinson's disease and tauopathy, when tau is abnormally hyperphosphorylated or altered in neurons of the central nervous system.

In the present specification, the term "neurodegenerative brain disease" may refer to any disease associated with neurodegenerative changes in the brain, and particularly may be used to comprehensively include any (brain) disease that can be caused by factors such as aggregation of amyloid-beta in the brain and/or brain nerve cells. In an embodiment, the neurodegenerative brain disease may be selected from the group consisting of dementia, Alzheimer's disease, Parkinson's disease, Huntington's disease, mild cognitive impairment, cerebral amyloid angiopathy, Down syndrome, amyloid stroke, systemic amyloidosis, Dutch type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia, but is not limited thereto. The neurodegenerative brain disease may include any disease that is caused by aggregation of amyloid-beta.

The term "normal" used herein refers to a subject not having the above-defined "neurodegenerative brain disease" among individuals of the same species and individuals to which the pharmaceutical composition is applied, or a brain tissue or cell (brain neurons) separated and/or cultured from the individuals.

In an embodiment, the TMEM176B, the polynucleotide encoding the same, or the agent for enhancing activity or expression of the TMEM176B and the polynucleotide may increase phagocytosis of amyloid-beta by a glial cell. In addition, the glial cell may be an ependymal cell, an oligodendrocyte, an astrocyte, or a microglia. Thus, the TMEM176B according to an embodiment, the polynucleotide encoding the same, or the agent for enhancing activity or expression of the TMEM176B and the polynucleotide can inhibit the aggregation and/or accumulation of amyloid-beta in the brain of an individual, and thus may be significantly used for diseases including neurodegenerative brain disease caused by the aggregation of amyloid-beta.

Another aspect provides a vector including the polynucleotide sequence encoding the TMEM176B.

The polynucleotide sequence encoding the TMEM176B may include, for example, a polynucleotide having at least 70 %, 80 %, 90 %, 95 %, 96 %, 97 %, 98 %, 99 %, or 100 % identity with the polynucleotide sequence encoding the SEQ ID NO: 1, wherein the protein encoded by the polynucleotide sequence may substantially retain the functional activity of the protein represented by SEQ ID NO: 1.

Another aspect provides a cell transformed with the vector.

Another aspect provides a pharmaceutical composition for preventing of treating neurodegenerative brain disease, including, as an active ingredient, the vector that includes the polynucleotide encoding theTMEM176B.

In an embodiment, the vector may be any one selected from the group consisting of a plasmid vector, a cosmid vector, a bacteriophage vector, an adenoviral vector, a retroviral vector, and an adeno-associated viral (AAV) vector, but is not limited thereto. In addition, the AAV vector may be any one selected from the group consisting of AAV1, AAV2, AAV3, AAV4, AAV5, AAV6, AAV7, AAV8, AAV9, AAV10, and AAV11, but is not limited thereto.

In detail, the present disclosure may provide an AAV vector including the polynucleotide encoding the TMEM176B. Preferably, the AAV vector may be in the form of AAV particles. Methods of preparing and modifying a viral vector and a particle thereof, such as those derived from the AAV, are well known in the art.

The AAV vector may include an AAV genome or a fragment or derivative thereof. The AAV genome is a polynucleotide sequence which encodes the functions needed for production of an AAV particle. These functions include those operating in the replication and packaging cycle for AAV in a host cell, including encapsidation of the AAV genome into an AAV particle. A naturally occurring AAV is replication-deficient and relies on the provision of helper functions in trans for completion of a replication and packaging cycle. The AAV genome may be in single-stranded form, either positive or negative-sense, or alternatively in double-stranded form. The use of a double-stranded form allows bypass of the DNA replication step in a target cell, and thus can accelerate transgene expression.

The AAV genome may be from any naturally derived serotype, isolate or clade of AAV. Thus, the AAV genome may be the full genome of a naturally occurring AAV. As is known in the art, AAVs occurring in nature may be classified according to various biological systems. In general, AAVs are referred to in terms of serotype thereof. A serotype corresponds to a variant subspecies of AAV, which owing to profile of expression of capsid surface antigens has a distinctive reactivity that can be used to distinguish it from other variant subspecies. Typically, a virus having a particular AAV serotype does not efficiently cross-react with neutralizing antibodies specific for any other AAV serotype. AAV serotypes include AAV1, AAV2, AAV3, AAV4, AAV5, AAV6, AAV7, AAV8, AAV9, AAV10 and AAV11, and also recombinant serotypes, such as Rec2 and Rec3, recently identified from primate brain. Any of these AAV serotypes may be used in the present invention.

The AAV may also be referred to in terms of clades or clones. It refers to the phylogenetic relationship of naturally derived AAVs, and typically to a phylogenetic group of AAVs that can be traced back to a common ancestor, and includes all descendants thereof. Additionally, the AAV may be referred to in terms of a specific isolate, i.e., a genetic isolate of a specific AAV found in nature. The term genetic isolate describes a population of AAVs that have undergone limited genetic mixing with other naturally occurring AAVs, thereby defining a recognizably distinct population at a genetic level.

Those skilled in the art can select an appropriate serotype, clade, clone, or isolate of AAV for use in the present disclosure on the basis of their common general knowledge.

The AAV vector may be prepared by introducing materials capable of generating a virus into a specific cell, whereas the expression vector including the nucleic acid according to the present disclosure is prepared by a method known in the art. For example, although not limited thereto, a virus may be introduced into a cell by transient transfection, microinjection, transduction, cell fusion, calcium phosphate precipitation, liposome-mediated transfection, DEAE dextran-mediated transfection, polybrene-mediated transfection, electroporation, gene gun, and other known methods of introducing nucleic acids into cells. For example, after an expression vector is prepared by inserting a TMEM176B-coding polynucleotide into an AAV, the vector is transduced into packaging cells, which are then cultured and filtered to obtain an AAV solution. Then, a TMEM176B gene may be introduced into cells by infecting cells, such as brain cells, neurons, and/or neural progenitors, using the AAV solution.

The vector including AAV may refer to a means for expressing a target gene in a host cell. The vector includes elements for the expression of a target gene, and may include a replication origin, a promoter, an operator, a transcription terminator, and the like. The vector may further include an appropriate enzymatic site (e.g., a restriction enzyme site) for introduction of a host cell into a genome and/or a selective marker for determining successful introduction into a host cell and/or a ribosome binding sites (RBS) for translation into proteins, an internal ribosome entry site (IRES ), and the like. The vector may further include a transcriptional regulation sequence (e.g., an enhancer and the like).

In the recombinant vector, the polynucleotide sequence encoding the protein may be operatively linked to a promoter. The term "operatively linked" refers to a functional linkage between a nucleotide expression regulatory sequence, such as a promoter sequence, and another nucleotide sequence, wherein the regulatory sequence regulates transcription and/or translation of the another nucleotide sequence.

The recombinant vector may be an expression vector capable of stably expressing TMEM176B a polynucleotide encoding the same in a host cell. For the expression vector, a conventional vector in the art used to express a foreign protein in plants, animals, or microorganisms may be used. The recombinant vector may be constructed through various methods known in the art.

In addition, the vector may refer to an expression vector for gene therapy. Accordingly, the viral vector may refer to a viral vector capable of delivering a therapeutic gene or a genetic material to a desired cell, tissue, and/or organ. The term "gene therapy" may refer to a method of normalizing functions by inserting a normal gene into a cell having genetic abnormality or providing a new function, so as to treat various genetic diseases caused by genetic abnormality. Accordingly, the recombinant vector of the present disclosure can prevent or treat neurodegenerative brain disease by expressing the TMEM176B or the polynucleotide encoding the same.

Another aspect provides a pharmaceutical composition for preventing or treating neurodegenerative brain disease, including, as an active ingredient, a cell transformed with the vector including the polynucleotide encoding the TMEM176B.

In the present specification, the term "transformation" refers to a change in the genetic properties of an organism by DNA given from the outside, and that is, may refer to a phenomenon in which a genetic trait changes when DNA, which is a type of nucleic acid extracted from a cell of a certain lineage of an organism, is introduced into a living cell of another lineage and enters the cell.

In an embodiment, the transformed cell may be a stem cell, a progenitor cell, or an animal cell, but is not limited thereto. In addition, the stem cell may specifically be an embryonic stem cell, an adult stem cell, or an induced pluripotent stem cell (iPS), but is not limited thereto. The stem cell may include a stem cell differentiated from the stem cell including all of a mesenchymal stem cell derived from an embryonic stem cell, a mesenchymal stem cell derived from an iPS, a neural stem cell derived from an iPS. In addition, the cell may be autologous or heterologous, or allogeneic or xenogenic.

The transformed cell of the present disclosure may prevent or treat neurodegenerative brain disease by expressing the TMEM176B or the polynucleotide encoding the same.

In the present specification, the term "treatment" is used in the sense of including all of alleviation or amelioration of pathological symptoms, reduction of a site of disease, delay or alleviation of disease progression, amelioration, alleviation, or stabilization of disease state or symptoms, partial or complete recovery, prolongation of survival, or other beneficial treatment results. The term "prevention" is used in the sense of including all mechanisms and/or effects of preventing the onset of, delaying the onset of, or reducing the frequency of the onset of a specific disease by acting on a subject that does not have the specific disease.

The term "pharmaceutical composition" may refer to a molecule or compound that confers some beneficial effects upon administration to a subject. Advantageous effects may include: enabling diagnostic decisions; ameliorating a disease, symptom, disorder, or condition; reducing or preventing the onset of a disease, symptom, disorder, or condition; and generally, responding to a disease, symptom, disorder, or condition.

The pharmaceutical composition may further include, in addition to the active ingredient, at least one adjuvant selected from the group consisting of a pharmaceutically acceptable carrier, an excipient, a diluent, a filler, an extender, a wetting agent, a disintegrant, an emulsifier (surfactant), a lubricant, a sweetening agent, a flavoring agent, a suspending agent, a preservative, and the like. The adjuvant may be appropriately adjusted according to a dosage form to which the pharmaceutical composition is applied, and may be used by selecting at least one from all adjuvants that can be commonly used in the field of pharmaceutics. In an embodiment, the pharmaceutically acceptable carrier is commonly used for drug formulation, and may be used by mixing at least one of saline, sterile water, Ringer's solution, buffered saline, dextrose solution, maltodextrin solution, glycerol, ethanol, liposome, and components thereof. Other conventional additives, such as an antioxidant, a buffer, and a bacteriostat, may be added as needed. In addition, by additionally adding a diluent, a dispersant, a surfactant, a binder, and a lubricant, the pharmaceutical composition may be formulated into an injectable formulation, such as an aqueous solution, a suspension, and an emulsion, a pill, a capsule, a granule, or a tablet. Also, to specifically act on a target organ, a target organ-specific antibody or other ligands may be combination with the carrier. Furthermore, by using appropriate methods in the art or a method disclosed it can be preferably formulated according to each disease or component using an appropriate method in the art or a method disclosed in Remington's document (e.g., Remington's Pharmaceutical Science (latest edition), the pharmaceutical composition may be preferably formulated depending on respective diseases or components.

An effective amount of the active ingredient or the pharmaceutical composition may be administered orally or parenterally during clinical administration, and may be used in the form of a general pharmaceutical formulation. Parenteral administration may refer to administration through a route, such as a rectal, intravenous, peritoneal, muscle, arterial, transdermal, nasal, inhale, ocular, or subcutaneous administration route, other than oral administration, and may include topical administration to a lesion site. For oral administration, the active ingredient in the pharmaceutical composition may be coated to prevent from decomposing in the stomach, or the pharmaceutical composition may be formulated into a dosage that is protectable from decomposition. When the pharmaceutical composition of the present disclosure is used as a medicine, at least one active ingredient that exhibits the same or similar functions may be further included.

In addition, in the specification, the term "active ingredient" may refer to a physiologically active substance that is a substance mentioned herein used to achieve the aforementioned pharmacological activity (e.g., treatment of neurodegenerative brain disease), and is distinguished from the substance mentioned herein administered alone, administered in combination with other substances, or additionally administered. That is, the TMEM176B or the polynucleotide encoding the same, the vector including the polynucleotide, the cell transformed with the vector including the polynucleotide, and the agent for enhancing activity or expression of the TMEM176B or the polynucleotide encoding the same may respectively be administered as a single active ingredient expecting a direct therapeutic effect on neurodegenerative brain disease.

The pharmaceutical composition may be in the form of a solution, a suspension, a syrup, or an emulsion in an aqueous or oily medium, or may be formulated in the form of a powder, a granule, a tablet, or a capsule. For formulation, a dispersant or a stabilizer may be additionally included. When formulating the pharmaceutical composition, a diluent or excipient, such as a filler, an extender, a binder, a wetting agent, a disintegrant, a surfactant, and the like that are commonly used may be used for the formulation. Formulations for the parenteral administration, a sterile aqueous solution, a non-aqueous solution, a suspension, an emulsion, a lyophilized formulation, and a suppository may be included. As a non-aqueous solvent and a suspension solvent, propylene glycol, polyethylene glycol, vegetable oil such as olive oil, and injectable ester such as ethyl oleate may be used. As a base agent for the suppository, witepsol, macrogol, tween 61, cacao butter, laurin butter, glycerol, gelatin, or the like may be used.

The pharmaceutical composition may be used by mixing with various pharmaceutically acceptable carriers, such as physiological saline or an organic solvent. To increase stability or absorptiveness of the pharmaceutical composition, a carbohydrate, such as glucose, sucrose, or dextran, an antioxidant, such as ascorbic acid or glutathione, a chelating agent, a low-molecular protein, or other stabilizers may be used as pharmaceuticals.

The pharmaceutical composition may be administered in a pharmaceutically effective amount. An administration dosage is not particularly limited, but may vary depending on absorption into the body, body weight, age, gender, and health condition of a patient, diet, administration time, administration method, excretion rate, and severity of disease. The pharmaceutical composition of the present disclosure is prepared in consideration of the effective amount range. A unit dosage form formulated thereby may be administered using a specialized dosing method according to the judgment of an expert who monitors or observes the drug administration or a personal demand as necessary, or may be administered several times at regular time intervals. A dose of the pharmaceutical composition may be in a range of 1 ug/kg/day to 1,000 mg/kg/day, but is not limited thereto. A daily or single dose may be formulated as one formulation in a unit dose form, formulated in an appropriate amount, or prepared by internalizing in a multi-dose container.

The subject may be a mammal, for example, a human, a cow, a horse, a pig, a dog, a sheep, a goat, or a cat. The subject may be an individual in need of treatment of neurodegenerative brain disease.

When the active ingredient of the present disclosure is a recombinant vector, an effective amount may be in a range of about 0.01 mg to about 500 mg, and more specifically, about 0.1 mg to about 300 mg. In the case of a recombinant virus including the recombinant vector, an effective amount may be specifically in a range of about 10³ IU to about 10¹² (10 PFU to 10¹⁰ PFU), and more specifically, about 10⁵ IU to about 10¹⁰. However, the effective amounts are not limited thereto.

When the active ingredient of the present disclosure is a cell, an effective amount may be in a range of about 10³ cells to 10⁸ cells, and more specifically, about 10⁴ cells to 10⁷ cells. However, the effective amounts are not limited thereto.

An effective dose of the composition of the present disclosure may be, per 1 kg of the body weight, in a range of about 0.05 mg/kg to about 12.5 mg/kg in the case of the recombinant vector, about 10⁷ virus particles to about 10¹¹ virus particles (10⁵ IU to 10⁹ IU)/kg in the case of the recombinant virus, and about 10³ cells/kg to about 10⁶ cells/kg. Specifically, the effective dose of the composition of the present disclosure may be in a range of about 0.1 mg/kg to 10 mg/kg in the case of the recombinant vector, about 10⁸ particles to about 10¹⁰ particles (10⁶ IU to 10⁸ IU)/kg in the case of the recombinant virus, and about 10² cells to about 10⁵ cells/kg in the case of the cell. The composition of the present disclosure may be administered 1 to 3 times a day. The constituents of the composition of the present disclosure are not necessarily limited thereto, and may vary depending on the condition and severity of a patient.

Another aspect provides a method of treating neurodegenerative brain disease, the method including administering to a subject with neurodegenerative brain disease a pharmaceutically effective amount of the TMEM176B or the polynucleotide encoding the same, the vector including the polynucleotide, the cell transformed with the vector including the polynucleotide, and the agent for enhancing activity or expression of the TMEM176B or the polynucleotide encoding the same.

Another aspect provides a method of preventing neurodegenerative brain disease, the method including administering to a subject a pharmaceutically effective amount of the TMEM176B or the polynucleotide encoding the same, the vector including the polynucleotide, the cell transformed with the vector including the polynucleotide, and the agent for enhancing activity or expression of the TMEM176B or the polynucleotide encoding the same.

Another aspect provides a method of inhibiting and/or disaggregating amyloid-beta aggregation, the method including administering to a subject in need of inhibition and/or disaggregation of the amyloid-beta aggregation a pharmaceutically effective amount of the TMEM176B or the polynucleotide encoding the same, the vector including the polynucleotide, the cell transformed with the vector including the polynucleotide, and the agent for enhancing activity or expression of the TMEM176B or the polynucleotide encoding the same. The method may further include, prior to the administering, identifying a subject in need of inhibition and/or disaggregation of the amyloid-beta aggregation.

Another aspect provides a method of disaggregating tau protein (and/or inhibiting aggregation) and/or inhibiting phosphorylation of tau protein, the method including administrating to a subject in need of disaggregation of tau protein (and/or inhibition of aggregation) and/or inhibition of phosphorylation of tau protein a pharmaceutically effective amount of the TMEM176B or the polynucleotide encoding the same, the vector including the polynucleotide, the cell transformed with the vector including the polynucleotide, and the agent for enhancing activity or expression of the TMEM176B or the polynucleotide encoding the same. The method may further include, prior to the administering, identifying a subject in need of disaggregation of tau protein (and/or inhibition of aggregation) and/or inhibition of phosphorylation of tau protein.

Another aspect provides a method of preventing and/or treating neurodegenerative brain disease, the method including administering, as an active ingredient, a pharmaceutically effective amount of the TMEM176B or the polynucleotide encoding the same, the vector including the polynucleotide, the cell transformed with the vector including the polynucleotide, and the agent for enhancing activity or expression of the TMEM176B or the polynucleotide encoding the same. The method may further include, prior to the administering, identifying a subject in need of prevention and/or treatment of neurodegenerative brain disease.

Another aspect provides a functional health food for preventing or improving neurodegenerative brain disease, the functional health food including, as an active ingredient, the agent for enhancing activity or expression of the TMEM176B or the polynucleotide encoding the same.

Another aspect provides a functional health food for preventing or improving cognitive impairment or improving memory, the functional health food including the agent for enhancing activity or expression of the TMEM176B or the polynucleotide encoding the same.

In the present specification, the term "cognitive" may refer to the mental activity or process of gaining knowledge and understanding through thinking, experience, and sensation. Here, processes such as knowledge, attention, memory and working memory, judgment and evaluation, reasoning and computation, problem solving and decision-making, and understanding and generation of language may be included. The cognitive impairment belongs to a category of mental health impairments that mainly affect learning, memory, perception, and problem solving, and may include amnesia, dementia and delirium.

The improvement of memory may refer to improvement of consequences of damage to neuroanatomical structures as well as of storage, retention, and recollection of memories. In addition, the improvement of memory may refer to improvement of memory impairments (including Alzheimer's disease) that can be progressive.

The functional health food may refer to a food prepared by using a raw material or ingredient having a nutrient that is easily deficient in daily meals or having a useful function for the human body (hereinafter, also referred to as 'functional raw material'). The functional health food may also refer to any food that helps to maintain health or to prevent and/or improve a certain disease or symptom, and a final product form thereof is not particularly limited. For example, the health functional food may be selected from the group consisting of various foods, beverage compositions, food additives, and the like, but is not limited thereto.

An amount of the active ingredient included in the health functional food is not particularly limited and appropriately depends on a type of food, desired use, or the like. For example, the amount may be in a e of about 0.0001 wt% to about 99 wt%, about 0.0001 wt% to about 95 wt%, about 0.0001 wt% to about 90 wt%, about 0.0001 wt% to about 80 wt%, about 0.0001 wt% to about 50 wt%, about 0.001 wt% to about 99 wt%, about 0.001 wt% to about 95 wt%, about 0.001 wt% to about 90 wt%, about 0.001 wt% to about 80 wt%, about 0.001 wt% to about 50 wt%, about 0.01 wt% to about 99 wt%, about 0.01 wt% to about 95 wt%, about 0.01 wt% to about 90 wt%, about 0.01 wt% to about 80 wt%, about 0.01 wt% to about 50 wt%, about 0.1 wt% to about 99 wt%, about 0.1 wt% to about 95 wt%, about 0.1 wt% to about 90 wt%, about 0.1 wt% to about 80 wt%, about 0.1 wt% to about 50 wt%, about 0.1 wt% to about30 wt%, about 0.1 wt% to about10 wt%, about 1 wt% to about 99 wt%, about 1 wt% to about 95 wt%, about 1 wt% to about 90 wt%, about 1 wt% to about 80 wt%, about 1 wt% to about 50 wt%, about 1 wt% to about 30 wt%, about 1 wt% to about 10 wt%, about 10 wt% to about 99 wt%, about 10 wt% to about 95 wt%, about 10 wt% to about 90 wt%, about 10 wt% to about 80 wt%, about 10 wt% to about 50 wt%, about 10 wt% to about 30 wt%, about 25 wt% to about 99 wt%, about 25 to 95 wt%, about 25 to 90 wt%, about 25 to 80 wt%, about 25 to 50 wt%, about 25 to 30 wt%, about 40 to 99 wt%, about 40 to 95 wt%, about 40 to 90 wt%, about 40 to 80 wt%, about 40 wt% to about 50 wt%, about 50 wt% to about 99 wt%, about 50 wt% to about 95 wt%, about 50 wt% to about 90 wt%, about 50 wt% to about 80 wt%, about 60 wt% to about 99 wt%, about 60 wt% to about 95 wt%, about 60 wt% to about 90 wt% %, or about 60 wt% to about 80 wt%, based on the total weight of the food. However, the amount is not limited thereto.

The health functional food may additionally include at least one selected from the group consisting of various nutrients, vitamins, minerals (electrolytes), flavoring agents, such as synthetic or natural flavoring agents, coloring agents, thickeners (cheese, chocolate, etc.), pectic acid or salts thereof, alginic acid or salts thereof, organic acids, protective colloidal thickeners, pH adjusters, stabilizers, preservatives, glycerin, alcohol, carbonation agents used in carbonated beverages, and the like. A ratio of these additives is generally selected from about 0.001 part by weight to about 20 parts by weight based on 100 parts by weight of the total health functional food, but is not limited thereto.

The technical terms and methods described for the present disclosure may be equally applied to the respective disclosures.

### ADVANTAGEOUS EFFECTS OF DISCLOSURE

According to a pharmaceutical composition for treating neurodegenerative brain disease, including, as an active ingredient, TMEM176B or a polynucleotide encoding the same, or an agent for enhancing activity or expression of the TMEM176B and the polynucleotide, wherein the TMEM176B increases phagocytosis of amyloid-beta by glial cells, the pharmaceutical composition not only promotes phagocytosis of amyloid-beta by astrocytes and reduces amyloid plaques, but also exhibits an effect of improving behavioral and cognitive functions in an animal model with Alzheimer's disease, thereby showing effectiveness for remarkable use in the prevention and/or treatment of various neurodegenerative brain diseases.

### BRIEF DESCRIPTION OF DRAWINGS

FIG. 1 is a graph showing results of measuring phagocytosis of astrocytes treated with TMEM176B inhibitor on FAM-labeled amyloid-beta;
FIG. 2 shows Western blot results and a graph confirming increased expression of TMEM176B in astrocytes into which a TMEM176B gene is introduced using an adeno-associated virus;
FIG. 3 is a graph confirming increased phagocytosis of astrocytes into which a TMEM176B gene is introduced using an adeno-associated virus;
FIG. 4 is a schematic diagram describing administration and evaluation methods for confirming a therapeutic effect of the TMEM176B-adeno-associated virus in a mouse model having Alzheimer's disease;
FIG. 5 is a photograph confirming reduction of plaques in the TMEM176B-adeno-associated virus administration group through amyloid-beta staining;
FIG. 6 shows graphs confirming reduction of plaques in the TMEM176B-adeno-associated virus administration group through amyloid-beta staining;
FIG. 7 is a graph confirming that the time to reach the platform is shortened in the TMEM176B-adeno-associated virus administration group through the water maze test; and
FIG. 8 shows graphs confirming that the cognitive function of the TMEM176B-adeno-associated virus administration group is improved in the behavior test after removal of the platform.

### MODE OF DISCLOSURE

Hereinafter, the present disclosure will be described in more detail with reference to Examples. However, these Examples are for illustrative purposes only, and the scope of the present disclosure is not intended to be limited by these Examples. It is apparent to those skilled in the art that the these Examples below may be modified without departing from the essential gist of the disclosure.

### Example 1. Confirmation of phagocytic activity of astrocyte by TMEM176B inhibition

It was confirmed how inhibition of TMEM176B affected phagocytic activity of an astrocyte.

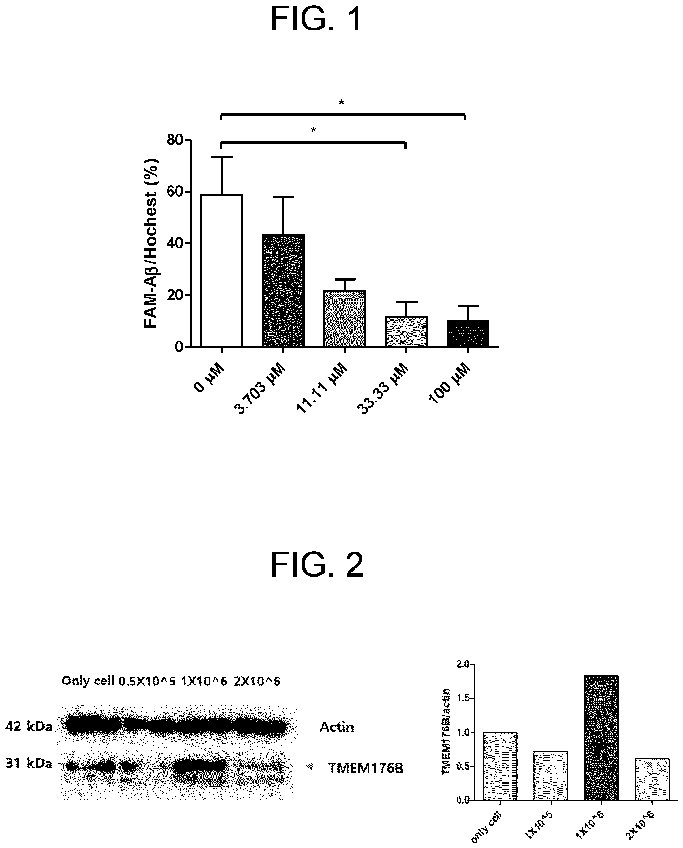

In detail, BAYK8644 (by Merck) was used as a TMEM176B inhibitor, a method of confirming phagocytic activity of an astrocyte (by Abm) after treatment with the inhibitor was performed as follows. The astrocyte was cultured in a 96-well black cell culture plate (by SPL) using a PriGrow IV medium containing 10 % FBS (by GIBCO), penicillin/streptomycin (by Thermo), human EGF 10 ng/mL (by Abm), and L-glutamine (by Abm), and incubation thereof was started at a density of 50,000 cells per well. Here, the TMEM176B inhibitor was treated at concentrations of 0 µM, 3.703 µM, 11.11 µM, 33.33 µM, and 100 µM, and the then the cells were stabilized by incubation at 37 °C in a 5% CO₂ incubator for 18 to 24 hours. Fluorescent FAM-labeled amyloid-beta was dissolved in cold PBS (by Anaspec) at a concentration of 5 µM, blocked from light, and stored in a refrigerator at 4 °C for 24 hours to prepare amyloid-beta in oligomers. After the stabilization was completed, the cells were treated with oligomeric amyloid-beta that was stored for 24 hours, and then incubated at 37 °C in a 5 % CO₂ incubator for 18 to 24 hours. After the incubation was completed, the culture solution was removed by suction, and 0.4 % trypan blue (by GIBCO) was added thereto and incubated for 1 minute at 37 °C in a 5 % CO₂ incubator. After 1 minute, trypan blue was removed by suction, and the phagocytized oligomeric amyloid-beta was measured at an excitation wavelength of 485 nm/emission wavelength of 535 nm using a microplate reader capable of measuring fluorescence. After the measurement was completed, a washing process was performed thereon once with PBS, and 50 µg/ml of Hoechst Dye 33342 (by abcam) was added and incubated at 37 °C in a 5 % CO₂ incubator for 30 minutes to stain the nuclei. After the incubation was completed, a washing process was performed thereon once with PBS, and the stained nuclei were measured at an excitation wavelength of 360 nm/emission wavelength of 465 nm. Results thereof are shown in FIG. 1 .

Consequently, as shown in FIG. 1 , it was confirmed that the treatment of astrocyte with the TMEM176B inhibitor decreased the phagocytosis function on the amyloid-beta oligomer. These results indicate that, if the TMEM176B were overexpressed or the activity thereof were enhanced, there would be a therapeutic effect on neurodegenerative brain diseases including Alzheimer's disease.

### Example 2. Confirmation of effect on astrocyte by enhancement of expression or activity of TMEM176B

By using a recombinant adeno-associated virus prepared to express a TMEM176B gene, overexpression of TMEM176B was induced in an astrocyte, and an effect of the overexpression was confirmed.

### <2-1> Preparation of TMEM176B-adeno-associated virus

By requesting Vigene Biosciences, a TMEM176B-adeno-associated virus was produced as a virus to which a CMV promoter and mouse-derived TMEM176B sequence were introduced using adeno-associated virus serotype 9.

### <2-2> Confirmation of expression of TMEM176B in astrocyte inoculated with TMEM176B-adeno-associated virus

An astrocyte was inoculated on a 12-well cell culture plate (by Corning) using a PriGrow IV medium (by Abm) containing 10 % FBS (by GIBCO), penicillin/streptomycin (by Thermo), and L-glutamin (by Abm), and L-glutamine (Abm) at a density of 100,000 cells per well. After 24 hours, to find out an appropriate inoculation concentration, an adeno-associated virus was inoculated thereon at concentrations of 0.5 genome copies (GC)/cell, 1 CG/cell, and 2×10⁶ GC/cell. 48 hours later, the infection of the cells was determined by a western blot. The western blot was performed using an anti-TMEM176B antibody (by Abclonal) and an HRP-conjugated anti-rabbit immunoglobulin G antibody (by Thermo), and results are shown in FIG. 2.

Consequently, as shown in FIG. 2, it was confirmed that the expression of the TMEM176B was approximately doubled at the inoculation concentration of 1×10⁶ GC/cell.

### <2-3> Confirmation of phagocytic activity of astrocyte with overexpressed TMEM176B

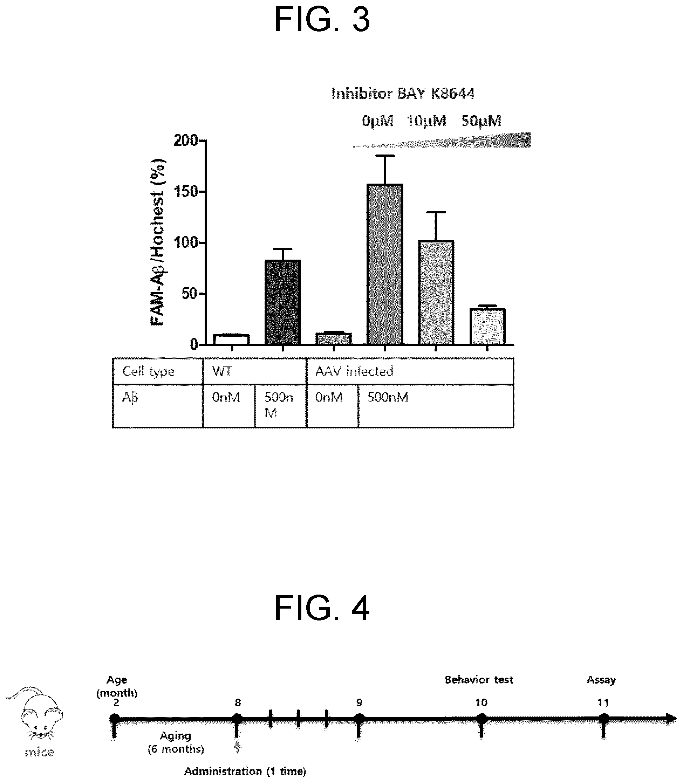

A TMEM176B-infected astrocyte and a normal astrocyte were separately cultured into wells of a 96-well black cell culture plate (by SPL) at a density of 50,000 cells per well, the plate using PriGrow IV medium containing 10 % FBS (by GIBCO), penicillin/streptomycin (by Thermo), 10 ng/mL of human EGF (by Abm), and L-glutamine (by Abm). Here, only the astrocytes inoculated with the TMEM176B-adeno-associated virus were treated with the TMEM176B inhibitor at concentrations of 0 µM, 10 µM, and 50 µM, and then stabilized by incubation at 37 °C in a 5% CO₂ incubator for 18 to 24 hours. FAM-labeled amyloid-beta (by Anaspec) was dissolved in cold PBS at a concentration of 5 µM, blocked from light, and stored in a refrigerator at 4 °C for 24 hours to prepare an oligomer. After the stabilization was completed, the cells were treated with oligomeric amyloid-beta stored for 24 hours, and then incubated at 37 °C in a 5 % CO₂ incubator for 18 to 24 hours. After the incubation was completed, the culture solution was removed, and 0.4 % trypan blue (by GIBCO) was added thereto and incubated for 1 minute at 37 °C in a 5 % CO₂ incubator. After 1 minute, trypan blue was removed, and the phagocytized oligomeric amyloid-beta was measured at an excitation wavelength of 485 nm/emission wavelength of 535 nm using a microplate reader capable of measuring fluorescence. After the measurement was completed, a washing process was performed thereon once with PBS, 50 µg/ml of Hoechst Dye 33342 (abcam) was added and incubated at 37 °C in a 5 % CO₂ incubator for 30 minutes to stain the nuclei. After the incubation was completed, a washing process was performed thereon once with PBS, and the stained nuclei were measured at an excitation wavelength of 360 nm/emission wavelength of 465 nm using a microplate reader capable of measuring fluorescence. Results thereof are shown in FIG. 3.

Consequently, as shown in FIG. 3, it was confirmed that phagocytosis was increased in the astrocytes inoculated with theTMEM176B-adeno-associated virus while phagocytosis was decreased in a concentration-dependent manner by the TMEM176B inhibitor. As the phagocytosis of amyloid-beta by the astrocytes was increased when the TMEM176B was overexpressed or the activity thereof was enhanced, the results above indicate that there is a therapeutic effect on neurodegenerative brain disease including Alzheimer's disease.

### Example 3. Confirmation of therapeutic effect by administration of TMEM176B-adeno-associated virus to mice with Alzheimer's disease

By introducing TMEM176B into an adeno-associated virus, the TMEM176B was set to be overexpressed upon in vivo infection. Mice with Alzheimer's disease were infected with the TMEM176B-adeno-associated virus, and it was confirmed whether aggregates of amyloid-beta were formed in the brain. Then, through behavior test using a water maze test, a therapeutic effect was confirmed .

### <3-1> Experiment to confirm TMEM176B efficacy using Alzheimer's disease mouse model

APP/PS1 Alzheimer's disease mouse models purchased from Jackson Lab were used, and the breeding and experiments were conducted at KPC. The received mice were acclimatized and aged for 6 months, and then administration of a TMEM176B-adeno-associated virus was started. During the acclimatization period, the mice were each bred one per cage and marked with ear-tacks. Labels were additionally attached to the cages to distinguish each mouse. When separating groups, the unique number for each mouse was used. After the group separation and initiation of the test, an individual identification card (group information, mouse number, sex, administration period, etc.) was attached to each breeding box. Each group consisted of 5 wild-type (WT) control groups, 8 APP/PS1 control groups, and 8 APP/PS1 TMEM176B administration groups. Animals were anesthetized using isoflurane inhalation anesthetic, and the head was fixed on a stereotaxic instrument. A Hamilton syringe was inserted into the brain location (AP: -0.58, ML: -1.25, DV: -2.1) for intracerebroventricular (ICV) administration, and 5 µl of a test substance was injected at a rate of 1 µl/min. After waiting for 5 minutes to 10 minutes to allow the test substance to diffuse to the surroundings, the syringe was withdrawn. After administered once at 32nd to 34th week, the behavior test was performed 8 weeks later. As shown in FIG. 4 , after administered once at the 8th month, the behavior test was performed 2 months later to confirm the efficacy of the TMEM176B.

FIG. 4 shows an experiment schedule for confirming the therapeutic effect of the TMEM176B-adeno-associated virus by using the Alzheimer's disease mouse models.

### <3-2> Confirmation of reduction in plaques through amyloid-beta staining

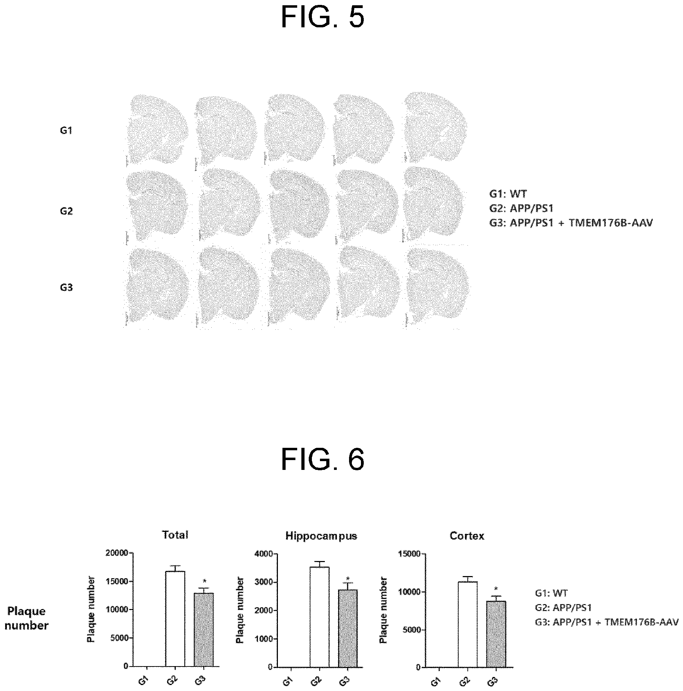

After completion of the treatment and behave test, the mice were sacrificed. Then, amyloid-beta plaques were detected from the wild-type (WT) control group (G1), the APP/PS1 control group (G2), and the APP/PS1 TMEM176 administration group (G3) for analysis. In detail, after fixing the brain in a 4% PFA solution and preparing a paraffin block, the paraffin block was cut using a microtome (HM340E, Thermo, USA) to a thickness of 4 µm and then attached on a slide. The slide was deparaffinized with xylene and rehydrated with ethanol (100 %-95 %-80 %-70 %) to completely remove paraffin. After treatment with 0.03% H₂O₂ for blocking of endogenous peroxidase, heat-mediated antigen retrieval was performed with a Tris/EDTA pH 9.0 buffer solution. The slide was incubated with a primary antibody (6E10, 1:2000) for 1 hour and a secondary antibody (Envision anti-rabbit HRP) for 30 minutes, developed with diaminobenzidine (DAB), and then counter-stained with Mayer's Hematoxylin. The analysis was performed using a confocal microscope, and the number and area of plaques were measured. Results are shown in FIGS. 5 and 6.

As a result, as shown in FIGS. 5 and 6, in the APP/PS1 TMEM176B administration group (G3), the number of plaques decreased in the whole brain, hippocampus, and cortex.

### <3-3> Confirmation of cognitive function improvement through Morris water maze test

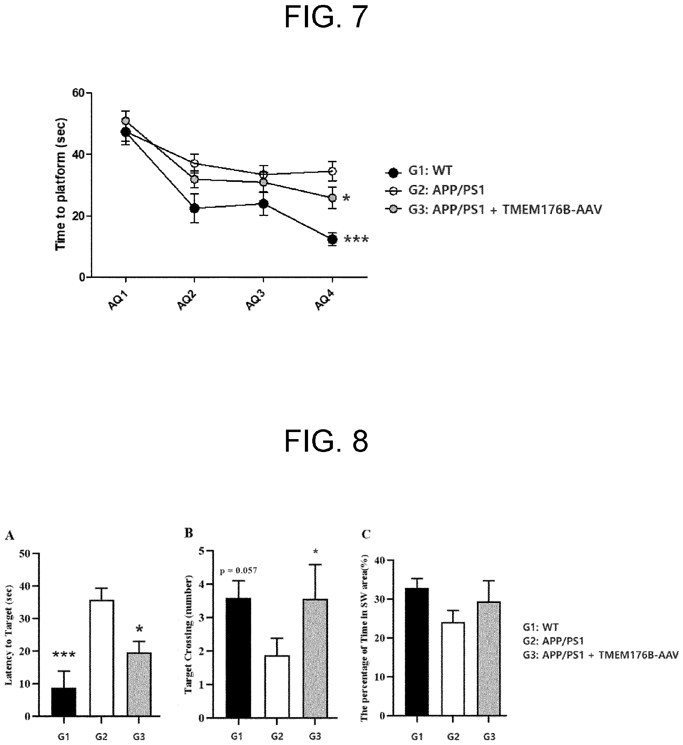

Based on the method devised by Morris, the water maze experiment was conducted. In detail, a stainless pool (a diameter of 90 cm and a height of 50 cm) was filled with water (having a temperature of 22±1 °C) to the level of 30 cm. A hidden platform (a diameter of 9 cm) was placed 1 cm below the water level. On the first day of evaluation, 3 to 4 training sessions were performed. The total swimming time per mouse was set to 60 seconds, and the mice that found the platform within 60 seconds were allowed to stay on the platform for 10 seconds to induce memory. The mice that failed to find the platform after 60 seconds were artificially guided to the platform and stayed on the platform for 10 seconds. Here, the escape time was set to 60 seconds. The acquisition test (AQ test) was conducted for 5 days, and the time to reach the platform was compared by date to verify the effectiveness of the test substance in relation to the cognitive function. On the first day, without recording the time to reach the platform, the training of reaching the platform using visual cues in four directions, i.e., east, west, north, and south directions, was conducted. For 4 days from the second day, the time to reach the platform was recorded (indicated by AQ1). Starting from each of the east, west, south, and north directions, the time to reach the platform was recorded. 4 data were recorded per mouse, and results are shown in FIG. 7.

Consequently, as shown in FIG. 7, in comparison with the G2 group by date, the G1 group showed a significant decrease in the time to reach the platform from AQ2 to AQ4 (last day) (AQ2, p=0.025; Aq3, p=0.018; Aq4, p<0.001). In addition, when comparing the G2 group with the G3 group which is the TMEM176B administration group, a significant difference was observed on the day of AQ4 (p=0.042).

Next, a probe test performed after removing the platform on the 6th day of the water maze test to measure the space perception ability (e.g., latency to target, target crossing numbers, percentage of time in SW area (%), etc.). The analysis was performed using a SMART VIDEO TRACKING Software (by Panlab, USA). A percentage of the time spent in the quadrant where the platform was located to the total swimming time (Percentage of time in SW area, %), the time required to reach a place where the platform was located (Latency to target, sec), and the number of times that the mice passed by the platform (Target Crossing, numbers) were compared. Results were calculated using a total of these three indicators, and the calculated results are shown in FIG. 8.

All data obtained in the above experiment were expressed as mean ± standard error mean (SEM), and all results were analyzed by one-way ANOVA using SPSS (by IBM SPSS Statistics, Version 22). The post-hoc test was performed by the least significant difference (LSD) test.

Referring to FIG. 8, as a result of comparing the G3 group with the G2 group, the G3 group showed a significant difference in the latency to target index (p=0.012), as well as in the target crossing (p=0.040).

The results above indicate that the TMEM176B according to an embodiment promotes phagocytosis of amyloid-beta by astrocytes, reduces amyloid plaques, and exhibits an effect of improving behavioral and cognitive functions in an animal model with Alzheimer's disease, thereby showing availability for remarkable use in the treatment of neurodegenerative brain disease.

## Claims

1. A pharmaceutical composition for preventing or treating neurodegenerative brain disease, comprising:
as an active ingredient, transmembrane protein 176B (TMEM176B), a polynucleotide encoding the same, or an agent for enhancing activity or expression of the TMEM176B or the polynucleotide.

2. The pharmaceutical composition of claim 1, wherein the TMEM176B comprises an amino acid sequence of SEQ ID NO: 1.

3. The pharmaceutical composition of claim 1, wherein the pharmaceutical composition is for administration to a subject in which a level of amyloid-beta or an aggregation level of amyloid-beta is higher than normal or at risk of being higher than normal, the normal not having neurodegenerative brain disease.

4. The pharmaceutical composition of claim 1, wherein the pharmaceutical composition is for administration to a subject in which phagocytosis activity of glial cells more dysfunctional than normal or at risk of being lower than normal, the normal not having neurodegenerative brain disease.

5. The pharmaceutical composition of claim 1, wherein the neurodegenerative brain disease is selected from the group consisting of dementia, Alzheimer's disease, Parkinson's disease, Huntington's disease, mild cognitive impairment, cerebral amyloid angiopathy, Down syndrome, amyloid stroke, systemic amyloidosis, Dutch type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

6. The pharmaceutical composition of claim 1, wherein the TMEM176B, the polynucleotide encoding the same, or the agent for enhancing activity or expression of the TMEM176B and the polynucleotide increases phagocytosis of amyloid-beta by a glial cell.

7. The pharmaceutical composition of claim 6, wherein the glial cell is an ependymal cell, an oligodendrocyte, an astrocyte, or a microglial cell.

8. A pharmaceutical composition for preventing or treating neurodegenerative brain disease, comprising
as an active ingredient, a vector comprising a polynucleotide encoding transmembrane protein 176B (TMEM176B).

9. The pharmaceutical composition of claim 8, wherein the vector is any one selected from the group consisting of a plasmid vector, a cosmid vector, a bacteriophage vector, an adenoviral vector, a retroviral vector, and an adeno-associated viral vector.

10. The pharmaceutical composition of claim 9, wherein the adeno-associated viral vector is any one selected from the group consisting of AAV1, AAV2, AAV3, AAV4, AAV5, AAV6, AAV7, AAV8, AAV9, AAV10, and AAV11.

11. A pharmaceutical composition for preventing or treating neurodegenerative brain disease, comprising
as an active ingredient, a cell transformed with a vector comprising a polynucleotide encoding transmembrane protein 176B (TMEM176B).

12. The pharmaceutical composition of claim 11, wherein the cell is any one selected from the group consisting of a stem cell, a progenitor cell, and an animal cell.

13. A function health food for preventing or ameliorating neurodegenerative brain disease, comprising
as an active ingredient, an agent for enhancing activity or expression of transmembrane protein 176B (TMEM176B) or a polynucleotide encoding the same.

14. A functional health food for preventing or ameliorating cognitive impairment or improving memory, comprising
as an active ingredient, an agent for enhancing activity or expression of transmembrane protein 176B (TMEM176B) or a polynucleotide encoding the same.

15. A method of preventing, ameliorating, or treating neurodegenerative brain disease, the method comprising
administering an effective amount of transmembrane protein 176B (TMEM176B), a polynucleotide encoding the same, or an agent for enhancing activity or expression of the TMEM176B or the polynucleotide, to a subject in need of the TMEM176B, the polynucleotide, or the agent.

16. Use of an effective amount of transmembrane protein 176B (TMEM176B), a polynucleotide encoding the same, or an agent for enhancing activity or expression of the TMEM176B or the polynucleotide for preparation of a composition for preventing, ameliorating, or treating neurodegenerative brain disease.
